# EUROPEAN PATENT SPECIFICATION

(11) **EP 3 962 471 B1**
(45) Date of publication and mention of the grant of the patent: **25.09.2024**
(21) Application number: 20730717.4
(22) Date of filing: 30.04.2020
(51) Int. Cl.: A61K 31/164, A61K 36/47, A61P 13/00, A61P 13/12

(54) **COMPOSITION FOR THE PREVENTION AND TREATMENT OF URINARY STONES**
ZUSAMMENSETZUNG ZUR VORBEUGUNG UND BEHANDLUNG VON HARNSTEINEN
COMPOSITION POUR LA PRÉVENTION ET LE TRAITEMENT DE CALCULS URINAIRES

(30) Priority: 02.05.2019 IT 201900006486
(43) Date of publication of application: 09.03.2022
(73) Proprietor: Neilos S.r.l., 80063 Piano di Sorrento (NA) (IT)
(72) Inventor: DI MAIO, Umberto, 80063 Piano di Sorrento (NA) (IT)
(74) Representative: Longoni, Alessandra
(86) International application number: PCT/IB2020/054093
(87) International publication number: WO 2020/222166

(56) References cited:
- PUCCI NIDIA D. ET AL: "Effect of phyllanthus niruri on metabolic parameters of patients with kidney stone: a perspective for disease prevention", INTERNATIONAL BRAZILIAN JOURNAL OF UROLOGY, vol. 44, no. 4, 10 March 2018 (2018-03-10), BR, pages 758 - 764, XP093024680, ISSN: 1677-5538, Retrieved from the Internet <URL:https://www.ncbi.nlm.nih.gov/pmc/articles/PMC6092661/pdf/1677-6119-ibju-44-04-0758.pdf> DOI: 10.1590/s1677-5538.ibju.2017.0521
- DANIELA IMPELLIZZERI ET AL: "Targeting inflammation: New therapeutic approaches in chronic kidney disease (CKD)", PHARMACOLOGICAL RESEARCH, vol. 81, 1 March 2014 (2014-03-01), AMSTERDAM, NL, pages 91 - 102, XP055544653, ISSN: 1043-6618, DOI: 10.1016/j.phrs.2014.02.007
- KUMADOH DORIS ET AL: "DOSAGE FORMS OF HERBAL MEDICINAL PRODUCTS AND THEIR STABILITY CONSIDERATIONS- An Overview", INNOVARE ACADEMIC SCIENCES, 1 August 2017 (2017-08-01), XP093024686, Retrieved from the Internet <URL:https://jcreview.com/admin/Uploads/Files/61c715d0548943.43560877.pdf> [retrieved on 20230216]
- YADAV R D ET AL: "Herbal plants used in the treatment of urolithiasis: A review", INTERNATIONAL JOURNAL OF PHARMACEUTICAL SCIENCES AND RESEARCH, SOCIETY OF PHARMACEUTICAL SCIENCES AND RESEARCH, IN, vol. 2, no. 6, 1 June 2011 (2011-06-01), pages 1412 - 1420, XP009174777, ISSN: 0975-8232
- ANONYMOUS: "Technical Data Report for CHANCA PIEDRA "Stone Breaker"", 1 January 2003 (2003-01-01), XP055446074, Retrieved from the Internet <URL:http://www.rain-tree.com/chanca-techreport.pdf> [retrieved on 20180130]
- MIRIAN A. BOIM ET AL: "Phyllanthus niruri as a promising alternative treatment for nephrolithiasis", INTERNATIONAL BRAZILIAN JOURNAL OF UROLOGY, vol. 36, no. 6, 1 December 2010 (2010-12-01), BR, pages 657 - 664, XP055159924, ISSN: 1677-5538, DOI: 10.1590/S1677-55382010000600002
- IUVONE TERESA ET AL: "Ultramicronized palmitoylethanolamide reduces viscerovisceral hyperalgesia in a rat model of endometriosis plus ureteral calculosis: role of mast cells", PAIN, LIPPINCOTT WILLIAMS & WILKINS - ELSEVIER, NL, vol. 157, no. 1, 1 January 2016 (2016-01-01), pages 80 - 91, XP009193869, ISSN: 1872-6623, DOI: 10.1097/J.PAIN.0000000000000220

## Description

### TECHNICAL FIELD

The present invention relates to a composition for the prevention and/or treatment of the symptomatology of urinary stones.

### BACKGROUND ART

Urinary stones or nephrolithiasis or urolithiasis is a pathological condition characterized by the presence of stones of varying sizes at any level of the urinary tract: calices, pelvis, ureter, bladder and urethra.

The stones usually form at the renal level and can subsequently migrate to the ureter where, by hindering the outflow of urine, they cause severe pain known as renal colic.

Urinary stones are not a disorder that affects the urinary tract only, but also entails a risk for the whole urinary system and in particular they can degenerate into chronic forms known as Chronic Kidney Disease (or CKD).

Some forms of nephrolithiasis, if not adequately treated, can lead up to advanced renal failure both owing to possible complications (for example, in infected stones), and owing to the underlying diseases that are the cause thereof (for example, cystinuria or hyperoxaluria primitive or hyperparathyroidism).

The epidemiology of urinary stones is strictly dependent on geographic factors; said differences are related to race, diet and climatic factors. In the socio-economically more advanced nations, the prevalence of urinary stones ranges between 4% and 20%, for an annual incidence of hospitalisations ranging from 0.04% to 0.30%. In Italy the prevalence of urinary stones is estimated to be from 6% to 9%, with an estimated incidence of about 100,000 new cases/year. Urinary stones are more frequent in white people, probably for reasons related to dietary habits. The male/female incidence ratio is close to 2/1, probably also given the role of female sex hormones in the decrease of urinary excretion of citrate. The male/female ratio in Italian cases stands at 1.5/1.

Calcium oxalate stones are more frequent in younger subjects, while mixed stones and uric acid stones tend to appear in older age.

There is numerous evidence in favour of the association of family factors with the development of urinary stones. The predisposition to an increased excretion of lithogenic solutes or to an increased tendency to crystallisation seems to come into play (defect of the inhibitors/increase of the promoters of the crystallisation process). Sometimes familiarity is however simply an epiphenomenon of the sharing of dietary or environmental factors among the various members of a family unit. Several studies have highlighted the correlation between animal protein intake and increased risk of urinary stones (to be related to the increase in urinary calcium excretion and the decrease in citrate excretion).

The association between two distinct measures of obesity - BMI (body mass index) and waist circumference - and an increased risk of urinary stones have been demonstrated. The intake of calcium and magnesium has been inversely correlated with the risk of stones (reduction of intestinal absorption of oxalate taken with the diet and consequent reduction of urinary excretion of the same). In the warmer regions (or in the warmer seasons in regions with a temperate climate) there is an increased incidence of urinary stones, in relation to the decreased urinary volume, secondary to the increased skin transpiration. The decreased volume implies an increase in urinary osmolarity, an increase in the concentration of calcium and oxalic acid and a decrease in urinary pH.

Renal colic is usually determined by an acute distension of the excretory pathway, secondary to the obstruction caused by urinary stones and is associated with a fairly typical symptomatic picture. Renal stones can appear with severe lower back pain, sometimes associated with distension of the urinary system, upstream of the stone, which arise slowly and progressively.

The typical symptoms of bladder stones are represented by intermittent and painful urination with terminal hematuria, with accentuation of the symptomatology at the end of urination. The distinctive feature of a stone obstructing the ureter or the renal pelvis is severe intermittent pain that radiates from the side to the groin or thigh. These are typically sharp pains lasting between 20 and 60 minutes, caused by the peristaltic contractions of the ureter which tries to expel the stone.

The link between the urinary, genital and gastrointestinal tract is at the basis of the irradiation of the pain in the gonads. Symptoms such as nausea and vomiting are also common. Postrenal azotemia and hydronephrosis can be observed after the obstruction of the urinary flow in one or both ureters.

Urinary stone is a solid aggregate of various shape and structure that is deposited inside the kidney or urinary tract. Its formation is usually due to the breakdown of a delicate balance maintained by renal function. The kidneys, in fact, for their function must on the one hand save water, on the other hand eliminate substances of various kinds with poor solubility, continuously adapting to different situations, of hydration, diet, climate, drug therapy, physical activity. In physiological conditions, the formation of stones does not occur due to the presence of substances in the urine that prevent the precipitation and crystallisation of calcium salts and due to the effect of others that bind calcium in soluble complexes. These mechanisms do not always guarantee an effective protection. If the urines become saturated with insoluble compounds, crystals are produced in a first phase which, by aggregating together, give rise to the stone.

Urinary stone can be distinguished into stones in calcium and non-calcium phase. Urinary stones in calcium phase includes among its forms the ones caused by precipitates of oxalate and/or calcium phosphate which is by far the most frequent form of urinary stones (incidence of 70-80%). The forms of non-calcium urinary stones are essentially constituted by uric stones characterized by the presence of uric acid or calcium urate (incidence of 5-15%), by infectious stones, caused by struvite (phosphate-ammonium-magnesium), struvite and apatite carbonate or ammonium urate and calcium phosphate (incidence of 10-15%) and by cystine stones, caused by cystine (incidence of 1-2%).

The initial diagnosis of urinary stones is based on a detailed anamnestic data collection so as to make all those risk factors related to the formation of stones and complications deriving from urolithiasis evident. Among the risk factors to be included are certainly:
- Any familiarity;
- Concomitant urological pathologies such as caliceal diverticulum, medullary sponge kidney, renal cyst, ureteral duplicity, urethral stenosis, horseshoe kidney, stenosis of the pelvic-ureteric junction, ureterocele, urinary tuberculosis;
- Non urological diseases that can cause urinary stones such as inflammatory bowel disease (Crohn's disease and Rectocolitis) and hyperparathyroidism;
- Use of drugs that can induce the formation of stones: among these there are drugs that are not very soluble that crystallize in the urine such as sulphonamides, cephalosporins, quinolones, anthrafenine, aluminum hydroxide, floctafenine, triamterene, sulfasalazine, guaiafenasin; drugs with metabolic effect such as carbonic anhydrase inhibitors, furosemide, magnesium hydroxide, nimesulide, laxatives, allopurinol, sodium bicarbonate or potassium;
- Lifestyle: obesity, sedentary lifestyle, prolonged work activity, reduced calcium intake, reduced frequency of water intake and increased skin transpiration.

The diagnostic process of urinary stones certainly includes an objective examination of the patient's physical state which helps to exclude all those pathologies that can appear with symptoms similar to a renal colic and in particular those that can jeopardize the patient's life. Furthermore, the urinalysis allows to detect parameters that can be of help for a first orientation in the diagnosis of stones of the urinary tract. The dosages of creatininemia, C-reactive protein, blood count with formula, sodemia and serum potassium, serum calcium and uric acid must be part of the initial assessment of the patient suffering from urinary stones, as well as urine culture with antibiogram. Also diagnostic imaging (urinary system x-ray, abdominal and pelvic ultrasound, echocolordoppler, urography, pyelography, renal scintigraphy, computed tomography and urographic magnetic resonance imaging) and more rare endoscopic diagnostics (urethrocystoscopy (UC) and ureterorenoscopy (URS)), represent a valid aid to confirm the diagnosis of urinary stones.

With regards to therapy, there are various drugs that are used to control pain in renal colic: NSAIDs, analgesic opiates, spasmolytic and other treatments such as ADH, acupuncture and locoregional anesthesia.

The use of drugs to speed up the spontaneous passage of stones through the ureter is referred to as "medical expulsive therapy". Several agents, including adrenergic alpha blockers (such as tamsulosin) and calcium channel blockers (such as nifedipine), have proven to be effective for this purpose. Alpha blockers appear to have a faster action, however, they appear effective only for stones between 4mm and 10mm in size. A combination of tamsulosin and a corticosteroid may be better than tamsulosin alone.

However, the need for new therapies that can be used in preventing and/or treating the symptomatology of urinary stones is felt in the art.

It is therefore an object of the present invention to provide a new pharmaceutical composition capable of preventing and/or treating the symptoms of urinary stones.

### DISCLOSURE OF INVENTION

The object of the present invention is achieved by a composition according to claim 1, by the use thereof according to claims 5 and 6, to a food supplement according to claim 7, a medical device according to claim 8 and a food for special medical purposes according to claim 9. Preferred embodiments of the invention form the subject matter of the dependent claims.

In particular, a composition comprising palmitoylethanolamide and an extract of *Phyllanthus niruri* is provided.

Palmitoylethanolamide (PEA), an amide of palmitic acid with ethanolamine, is a molecule found in many foods of the common diet such as eggs, soy, tomatoes, peas and peanuts. It is also produced by many cells of the organism of mammals and is particularly abundant in brain tissues. PEA and other acylethanolamides (NAE) are produced starting from phospholipid precursors found in the plasma membrane. PEA is synthesized following stimuli capable of evoking an immune response. Part of the lipid mediators responsible for the resolution of inflammation is an active and coordinated programme that takes place already in the first hours after the onset of the inflammatory response. The anti-inflammatory or pro-resolving mediators actively counteract the action of pro-inflammatory mediators. At the cellular level, this results in the stimulation of phagocytosis of apoptotic leukocytes, the promotion of apoptosis of damaged cells up to neuroprotection. At the clinical level, the endogenous anti-inflammatory and pro-resolving action results in pain reduction. Under pathological conditions characterized by cellular damage and inflammatory processes, the endogenous PEA levels are altered; said alterations in PEA levels in the conditions accompanied by inflammatory processes suggest that the exogenous supply of the molecule may favour and accelerate the process of resolution of the inflammation and restoration of the tissue homeostasis.

PEA was considered to be an endocannabinoid (eCB), since it was initially proposed as a cannabinoid 2 receptor agonist (CB2). Currently, several mechanisms have been proposed to explain the anti-inflammatory and analgesic effects of PEA, including: (i) the activation of a cell surface receptor (i.e. CB2-like or, alternatively, the orphan receptor GPR55) or otherwise of a nuclear receptor from the family of the PPAR receptors (receptors activated by peroxisome proliferators); (ii) a down-modulation of mast cell hyperactivity; (iii) an action as an "entourage" compound, i.e. capable of causing an increase in the activity of the endocannabinoids on their receptors and/or the inhibition of the degradation of the same. The chemical similarity of PEA with the endocannabinoid anandamide and the evidence on the interactions between PEA and the system of endocannabinoids have favoured the carrying out of a series of pharmacological studies, the results thereof confirmed the efficacy of PEA in promoting the resolution processes of inflammation under experimental conditions characterized by a high involvement of mast cells. In fact, growing evidence indicates that the exogenous administration of PEA has a key role in promoting inflammation resolution processes, with consequent reduction of pain.

A study has shown that PEA orally administered to rats (10 mg/kg) is able to inhibit mast cell degranulation and peripheral inflammation, also reducing carrageenan-induced oedema in the rat paw, which effects are accompanied by changes in the production of nitric oxide by macrophages and in the expression of pro-inflammatory proteins, such as inducible nitric oxide synthase (iNOS) and cyclooxygenase-2 (COX-2). A study conducted in mice clearly showed that PEA significantly attenuated the degree of renal dysfunction, lesions and inflammation caused by ischemia-reperfusion injury and these positive effects were at least partially dependent on the PPAR-α cascade. In particular, the treatment with PEA also significantly reduced lipid peroxidation and nitrotyrosine expression in the kidney, probably in part correlated to the reduced neutrophil recruitment. A study gathers 21 clinical studies, of which 16 enroll 20 to 636 patients and five studies are pilot cases. In clinical studies, PEA has been used for periods ranging from 14 to 120 days, and doses range from 300 mg to 1200 mg per day. The form of administration of PEA was in most cases in oral tablets except for the occasional use of sublingual formulations (sachets) and the most common form of assessment was the visual analogue scale (VAS), where the patient makes a subjective assessment of his/her pain level on a 10 cm line where the left side does not represent pain, and the right side represents the worst imaginable pain. With one exception, all available clinical studies reported a significant reduction in pain intensity and an almost total absence of side effects.

Moreover, Iuvone et al, Pain, (2016), vol. 157, no. 1, pages 80 - 91, describes the use of palmitoylethanolamide for the treatment of pain caused by the combination of endometriosis and urethral calculosis.

*Phyllanthus niruri,* popularly known as "stonebreaker" is a plant belonging to the Euphorbiaceae family and is used in Brazilian folk medicine for patients with urolithiasis. More than 50 compounds have been identified in the *Phyllanthus niruri,* including alkaloids, flavonoids, lignans and triterpenes. Among said substances, triterpenes have shown the ability to inhibit calcium oxalate-induced cytotoxicity and to reduce the excretion of the constituents of the stone and of the markers of crystal deposition in the kidneys. In addition, the methanolic extract of the leaves of *Phyllanthus niruri,* containing substances such as lignans and phylanthine, showed uricosuric activity (increased uric acid excretion with the urine) in hyperuricemic rats. Another study shows that alkaloids extracted from plants of the Phyllanthus genus exhibit antispasmodic activity that induces relaxation of smooth muscle, mostly highlighted in the urinary tract, which would facilitate the elimination of the stone. The effect of the aqueous extract of *Phyllanthus niruri* on the crystallisation process of calcium oxalate in human urine has been studied in a precipitation model *in vitro* of calcium oxalate in human urine. It has been observed that the pre-incubation of human urine with *Phyllanthus niruri* did not inhibit the precipitation of calcium oxalate particles, but the crystals obtained were proportionally smaller than those in urine samples without *Phyllanthus niruri.* Furthermore, it was observed that after 24 hours, the crystals in the untreated urines formed large agglomerations, while the crystals in the urine treated with *Phyllanthus niruri* remained dispersed. In addition to intervening on the crystallisation of calcium oxalate, it was noted that the methanolic extract of *Phyllanthus niruri* showed *in vitro* an inhibitory activity of the enzyme xanthine oxidase, a property attributed to compounds such as flavonoids, polyphenols and tannins. An *in vivo* study performed on adult albino rats and on mice shows how the spray-dried standardized extract of *Phyllanthus niruri,* exert an anti-inflammatory and analgesic action; in this study, the intraperitoneal administration at 100, 200, 800 or 1600 mg/kg of the extract of Phyllanthus demonstrated to reduce the vascular response in the inflammatory process of the oedema induced in the rat paw by 1% carrageenan. The same extract administered orally at 100 or 200 mg/kg also inhibited the leukocyte migration on the inflammation site induced by 3% thioglycolate. The same study also boasted the analgesic effects of aforesaid extract which, administered intraperitoneally at 100 or 200 mg/kg in rats, showed peripheral analgesic effects through the Randall-Sellitto test and a significant analgesic activity at central level through the hot plate and the tail flick test. A clinical study that deals with the effects of *Phyllanthus niruri* administered in the form of a tea, did not show any clinical or biochemical adverse effects (cardiovascular, renal, hepatic or neurological) even at high doses, with excellent tolerability in healthy volunteers. In addition, the consumption of tea in the same dose by patients with nephrolithiasis for a period of 3 months, led to an apparent increase in the elimination of stones compared to patients drinking placebo. Said results have probably been attributed to the antispasmodic and relaxing effects of *Phyllanthus niruri* on the ureteral muscle, which facilitates the elimination of stones. Another study showed that patients undergoing extracorporeal shock wave lithotripsy and treated with *Phyllanthus niruri* during the 3-month period had a lower incidence of residual stone fragments, mainly those in a lower calyceal position compared to untreated patients. According to said investigators, the efficacy and the lack of adverse events of Phyllanthus help to improve the overall results after extracorporeal shock wave lithotripsy and this might be useful as an alternative or additional therapy in the treatment of urolithiasis.

Moreover, Mirian et al, International Brazilian Journal of Urology, vol. 36, no.6, (2010), 657-664) describes the effects of the plant *Phyllanthus niruri* on certain stages of kidney stone formation and its internalisation by tubular cells.

In an embodiment of the invention, the composition comprises 10 to 5000 mg of palmitoylethanolamide and 10 to 5000 mg of an extract of *Phyllanthus niruri,* it preferably comprises 150 to 800 mg of palmitoylethanolamide and 20 to 500 mg of an extract of *Phyllanthus niruri,* it more preferably comprises 300 to 600 mg of palmitoylethanolamide and 50 to 450 mg of an extract of *Phyllanthus niruri.*

The composition of the invention can be used as medicament. Advantageously, the composition can be used in the preparation of a food supplement, a medical device and a food for special medical purposes.

Advantageously, the inventors have shown how the association of palmitoylethanolamide and an extract of *Phyllanthus niruri* leads to a synergistic action of the two components that allows obtaining simultaneously an analgesic and an anti-inflammatory effect useful in preventing and treating urinary stones, which is often accompanied by pain in the lumbar region and renal inflammation due to the obstruction of the urinary pathway due to the presence of the stone.

Thanks to Palmitoylethanolamide, the present invention constitutes a valid aid for the analgesic action, exerted by enhancing the activity of the endocannabinoids on their receptors and through the regulation of the nuclear receptor of the PPAR family (receptors activated by peroxisome proliferators) and for the anti-inflammatory action that PEA exerts by down-modulating the mast cell degranulation. Thanks to *Phyllanthus niruri* aforesaid activities are enhanced, in fact it acts on the inflammation by inhibiting the leukocyte migration and on pain thanks to its antispasmodic and relaxing activity which in case of stones makes expulsion less painful. The efficacy of the composition, object of the present invention, has been assessed according to experimental protocols known to those skilled in the art. In particular, for the assessment of the various activities of the composition of the present invention, *in vitro* and/or *in vivo* assays known in the scientific literature can be used.

To demonstrate the analgesic efficacy of the composition of the present invention, particularly interesting for the prevention and/or treatment of urinary stones, *in vitro* assays proves to be suitable such as those using rabbit jejunum or Trendelenburg's guinea-pig ileum.

To demonstrate instead the anti-inflammatory efficacy of the composition of the present invention, *in vitro* assays assessing the ability to inhibit the release of inflammatory cytokines such as IL-1, IL-6 and TNF-α and to inhibit the expression of enzymes such as COX-2 and metalloproteases-13 induced by IL-1β in primary human cell cultures prove to be suitable (e.g. macrophages, chondrocytes and fibroblasts).

Further characteristics of the present invention will become clear from the following description of some merely illustrative and non-limiting examples.

### BEST MODE FOR CARRYING OUT THE INVENTION

### Example 1

The composition of the present invention can be found in pharmaceutical forms for oral administration in which the active ingredients are present in the following concentrations:

### EXAMPLE 1A

| Active ingredient | Quantity per single unit |
|---|---|
| PEA | 400 mg |
| Phyllanthus niruri extract | 200 mg |

| | |
|---|---|
| Pharmaceutical form: tablets | |

### EXAMPLE 1B

| Active ingredient | Quantity |
|---|---|
| PEA | 300 mg |
| Phyllanthus niruri extract | 150 mg |

| | |
|---|---|
| Pharmaceutical form: capsules | |

### EXAMPLE 1C

| Active ingredient | Quantity |
|---|---|
| PEA | 400 mg |
| Phyllanthus niruri extract | 300 mg |

| | |
|---|---|
| Pharmaceutical form: coated tablet | |

### EXAMPLE 1D

| Active ingredient | Quantity |
|---|---|
| PEA | 600 mg |
| Phyllanthus niruri extract | 400 mg |

| | |
|---|---|
| Pharmaceutical form: sachets | |

### EXAMPLE 1E

| Active ingredient | Quantity |
|---|---|
| PEA | 400 mg |
| Phyllanthus niruri extract | 450 mg |

| | |
|---|---|
| Pharmaceutical form: sachets | |

### Example 1F

| Active ingredient | Quantity |
|---|---|
| PEA | 300 mg |
| Phyllanthus niruri extract | 200 mg |

| | |
|---|---|
| Pharmaceutical form: sachets | |

### Efficacy test on rats

An *in vivo* model aims to test the composition with regards to its anti-inflammatory effect which manages to restore the kidney damage induced by the formation of stones.

The recruited rats were randomly divided into five groups. Group I was used as a vehicle and was maintained on a regular basis of food and water ad libitum and received a carboxymethylcellulose solution (e.g. 5 ml/kg po). All the remaining groups were treated with compounds capable of accelerating lithiasis (e.g. ethylene glycol and ammonium chloride) contained in the drinking water ad libitum for three days and were subsequently treated by administering only ethylene glycol or other similar compound for 25 days, to induce hyperoxaluria.

Group II simultaneously received a carboxymethylcellulose solution and was used as a control. Group III received PEA, group IV received an extract of *Phyllanthus niruri* and group V received both compounds from the first to the twenty-eighth day of stone induction respectively.

Both the single components and the composition according to the invention were suspended in water using a carboxymethylcellulose solution or other similar compound and were administered orally once a day (e.g. 5 ml/kg body weight). The kidney tissue of the rats in which the stones are induced, shows significant histological changes with interstitial fibrosis accompanied by dense infiltration of eosinophils, therefore a strong inflammatory reaction as well as a significant accumulation of calcium oxalate in the renal tubules, with consequent increase in urinary volume. Said model aims to demonstrate how the treatment with both compounds can restore the inflammatory damage to the kidney tissue and normalise oxalate and calcium levels, significantly compared to the control group and to the single components.

## Claims

1. A composition comprising palmitoylethanolamide and an extract of *Phyllanthus niruri.*

2. The composition according to claim 1 **characterized in that** it comprises 10 to 5000 mg of palmitoylethanolamide and 10 to 5000 mg of an extract of *Phyllanthus niruri.*

3. The composition according to claim 2 **characterized in that** it comprises 150 to 800 mg of palmitoylethanolamide and 20 to 500 mg of an extract of *Phyllanthus niruri.*

4. The composition according to claim 3 **characterized in that** it comprises 300 to 600 mg of palmitoylethanolamide and 50 to 450 mg of an extract of *Phyllanthus niruri.*

5. The composition according to any one of the preceding claims for use as medicament.

6. The composition according to any one of the claims from 1 to 5 for use in preventing or treating the symptomatology of urinary stones.

7. Food supplement comprising the composition according to any one of claims 1-4.

8. Medical device comprising the composition according to any one of claims 1-4.

9. Food for special medical purposes comprising the composition according to any one of claims 1-4.

## Patentansprüche

1. Zusammensetzung, die Palmitoylethanolamid und einen Extrakt aus *Phyllanthus niruri* umfasst.

2. Zusammensetzung gemäß Anspruch 1, **dadurch gekennzeichnet, dass** sie 10 bis 5000 mg Palmitoylethanolamid und 10 bis 5000 mg eines Extrakts von *Phyllanthus niruri* umfasst.

3. Zusammensetzung gemäß Anspruch 2, **dadurch gekennzeichnet, dass** sie 150 bis 800 mg Palmitoylethanolamid und 20 bis 500 mg eines Extrakts von *Phyllanthus niruri* umfasst.

4. Zusammensetzung gemäß Anspruch 3, **dadurch gekennzeichnet, dass** sie 300 bis 600 mg Palmitoylethanolamid und 50 bis 450 mg eines Extrakts von *Phyllanthus niruri* umfasst.

5. Zusammensetzung gemäß einem der vorhergehenden Ansprüche zur Verwendung als ein Medikament.

6. Zusammensetzung gemäß einem der Ansprüche 1 bis 5 zur Verwendung bei der Vorbeugung oder Behandlung der Symptomatologie von Harnsteinen.

7. Nahrungsergänzungsmittel, das die Zusammensetzung gemäß einem der Ansprüche 1 bis 4 umfasst.

8. Medizinische Vorrichtung, die die Zusammensetzung gemäß einem der Ansprüche 1 bis 4 umfasst.

9. Nahrungsmittel für besondere medizinische Zwecke, das die Zusammensetzung gemäß einem der Ansprüche 1 bis 4 umfasst.

## Revendications

1. Composition comprenant du palmitoyléthanolamide et un extrait de *Phyllanthus niruri.*

2. Composition selon la revendication 1 **caractérisée en ce qu'**elle comprend 10 à 5000 mg de palmitoyléthanolamide et 10 à 5000 mg d'un extrait de *Phyllanthus niruri.*

3. Composition selon la revendication 2 **caractérisée en ce qu'**elle comprend 150 à 800 mg de palmitoyléthanolamide et 20 à 500 mg d'un extrait de *Phyllanthus niruri.*

4. Composition selon la revendication 3 **caractérisée en ce qu'**elle comprend 300 à 600 mg de palmitoyléthanolamide et 50 à 450 mg d'un extrait de *Phyllanthus niruri.*

5. Composition selon l'une quelconque des revendications précédentes destinée à être utilisée en tant que médicament.

6. Composition selon l'une quelconque des revendications 1 à 5 destinée à être utilisée pour la prévention ou le traitement de la symptomatologie des calculs urinaires.

7. Complément alimentaire comprenant la composition selon l'une quelconque des revendications 1 à 4.

8. Dispositif médical comprenant la composition selon l'une quelconque des revendications 1 à 4.

9. Aliment destiné à des fins médicales spéciales comprenant la composition selon l'une quelconque des revendications 1 à 4.
